# EUROPEAN PATENT APPLICATION

(11) **EP 1 582 161 A1**
(43) Date of publication of application: **05.10.2005**
(21) Application number: 04007787.7
(22) Date of filing: 31.03.2004
(51) Int. Cl.: A61B 17/72

(54) **Intramedullary nail provided with expansion fixing means operated by one or more driving elements**

(71) Applicant: ORTHOFIX INTERNATIONAL B.V., 1071 Amsterdam (NL)
(72) Inventor: Coati, Michele, 37029 San Pietro in Cariano (Verona) (IT); Marazzi, Giancarlo, 21047 Saronno (Varese) (IT); Marini, Graziano, 37060 Castel D'Azzano (Verona) (IT); Rossi, Graziano, 37139 Verona (IT); Rossi, Luigi, 37019 Peschiera del Garda (Verona) (IT); Venturini, Daniele, 37064 Povegliano Veronese (Verona) (IT)
(74) Representative: Ferreccio, Rinaldo

(57) **Abstract**

An intramedullary nail (10, 110, 210) suitable for insertion in a fractured elongate bone (12, 112, 212) and for an unusually simple fixation therein comprises a substantially straight stem (14, 114, 214) extending between a proximal end (16, 116, 216) and a distal end (18, 118, 218), said stem (14, 114, 214) comprising, near each of said proximal (16, 116, 216) and distal (18, 118, 218) ends, expansion means (20, 120, 220; 21, 121, 221) for the nail fixation to the bone, said expansion means (20, 120, 220; 21, 121, 221) being operated by at least one driving element (30, 130, 230), interposed along the axis of the stem (14, 114, 214) between said expansion means (20, 120, 220) of said proximal end (16, 116, 216) and said expansion means (21, 121, 221) of said distal end (18, 118, 218) and realised with a shape-memory material.

## Description

### Field of application

The present invention relates in its more general aspect to an intramedullary nail suitable for insertion in a fractured elongate bone and an application method of said nail in said bone.

In particular, the invention relates to an intramedullary nail suitable for insertion in a fractured elongate bone, such as a femur or a tibia, comprising a substantially straight stem extending between a proximal end and a distal end.

### Prior art

Intramedullary nails are known, which, during a surgical operation, are inserted in a fractured elongate bone and fixed therein, in order to reconstruct the original bone shape and in the meantime to recover the bone solidity, so that callus regeneration mechanisms can correctly occur.

The stems of these intramedullary nails are generally cylinder-shaped and they can be both solid and hollow.

In order to fix the intramedullary nail to the bone portions to be reconstructed, two offset holes are usually provided on the nail, having axes lying on parallel planes and extending diametrically across the stem, in correspondence with the nail distal end, and two offset holes having the same size, having axes not necessarily lying on parallel planes, in correspondence with the nail proximal end. Said holes are suitable for housing bone screws, which are inserted, after a convenient bone drilling, in the bone, with the subsequent fixation of the intramedullary nail to the bone portions.

Although advantageous under different points of view, intramedullary nails being structured as above schematically described have known drawbacks mainly occurring when bone drillings are to be performed for bone screw insertion. This step is particularly critical since it is known that a good nail fastening essentially depends on the correct realisation of these bone drillings, obviously made in correspondence with the holes of the inserted intramedullary nail.

However the precise location of the intramedullary nail holes is made difficult by the fact that the holes are no more visible, being the nail inserted in the bone. It is then worth underlining a further location problem, i.e the fact that the intramedullary nail can be, when being inserted, slightly bent, so that the holes at the nail distal end are no more, with respect to the proximal end, in the same position as before installing the nail.

The traditional technique for locating the holes of an inserted intramedullary nail provides the use of X rays, involving however the well known risks of cumulative exposure of the operating staff, besides being quite awkward during the surgical operation.

Specific mechanical devices have thus been studied and realised, such as for example the one described in the European patent no. EP 772 420 in the name of the same Applicant.

These devices do not require the use of X rays, but they have the drawback of requiring several operational steps, all to be performed quite carefully and precisely.

### Summary of the invention

The problem underlying the present invention is to provide an intramedullary nail suitable for insertion in a fractured elongate bone, capable to meet the above-mentioned requirement, meanwhile overcoming, in a simple and effective way, all the drawbacks mentioned with reference to the prior art.

This problem is solved, according to the present invention, by an intramedullary nail suitable for insertion in a fractured elongate bone, as above described and characterised in that said stem comprises, near each of said proximal and distal ends, expansion means for the nail fixation to the bone, said expansion means being operated by at least one driving element, interposed along the stem axis between said expansion means of said proximal end and said expansion means of said distal end and realised with a shape-memory material.

Further features and the advantages of the intramedullary nail suitable for insertion in a fractured elongate bone, as well as of the application method of said nail in said fractured bone, according to the present invention, will be apparent from the following description of an embodiment thereof made with reference to the attached drawings, given by way of non-limiting example.

### Brief description of the drawings

Figure 1 is a schematic perspective view of a first embodiment of an intramedullary nail, suitable for insertion in a fractured elongate bone, according to the present invention.
Figure 2 is a schematic perspective view of a second embodiment of an intramedullary nail, suitable for insertion in a fractured elongate bone, according to the present invention.
Figure 3 is a schematic enlarged-scale perspective view of a portion of the nail of figure 2.
Figure 4 is a schematic perspective view of a third embodiment of an intramedullary nail, suitable for insertion in a fractured elongate bone, according to the present invention.
Figure 5 is a schematic enlarged-scale perspective view of a portion of the nail of figure 4.
Figure 6 is a schematic enlarged-scale perspective view of a further portion of the nail of figure 4.
Figure 7 is a schematic enlarged-scale perspective view of a component of the nail of figure 4.

### Detailed description

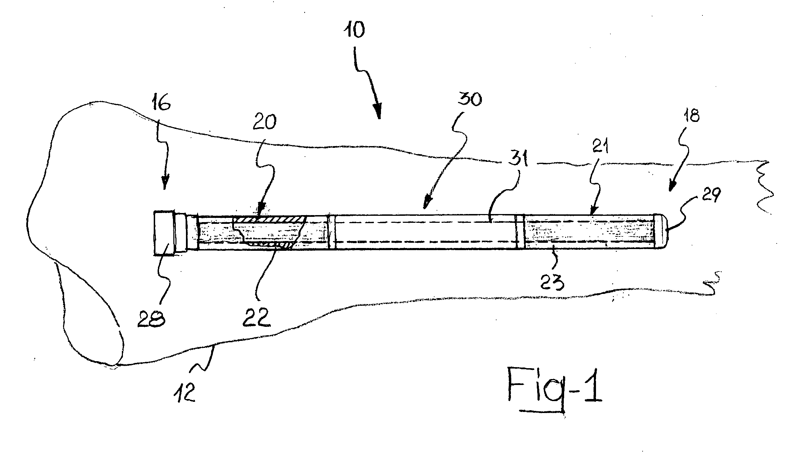

Referring first to figure 1, an intramedullary nail according to the present invention is shown and globally indicated with 10, suitable for insertion in a fractured elongate bone 12, such as for example a femur or a tibia.

The nail 10 comprises a substantially straight stem 14 extending between a proximal end 16 and a distal end 18.

The stem 14 is preferably composed of a cylindrical tube, realised for example with a titanium alloy. The stem 14, according to an aspect of the present invention, comprises, near said proximal 16 and distal 18 ends, expansion means 20 and 21 respectively for the fixation of the nail 10 to the bone 12, said expansion means 20 and 21 being operated by at least one driving element 30, interposed between said expansion means 20 of said proximal end 16 and said expansion means 21 of said distal end 18 and realised with a shape-memory material.

Shape memory material means a material having a given initial starting shape and taking, under predetermined external conditions (for example a temperature rise and/or drop) or undergoing a predetermined activation condition, i.e. after a so-called "material instruction" step, a given new shape.

Known shape-memory material, which are suitable for use in the present invention, are, for instance, certain nickel-titanium alloys.

The invention is based on the following principle: subjecting an element realised in a shape memory material, having a predetermined initial shape at rest, to said so-called "material instruction" step (for example to a predetermined temperature variation), said element takes a shape being maintained as long as the "instruction" step effect persists; said shape, being different from the initial shape, can be called transient shape and it is temporary or unstable. When the "instruction" step effect stops, the element leaves said transient shape and, coming back towards the initial shape, takes a working shape. Depending on the type of material and on the working temperature, in the working shape, said material can arrive at the initial shape or it can go further on the initial shape, arriving at a final shape. It is worth pointing out that the more this final shape is far from the initial shape, the more the available shape memory energy of the material is high.

The invention has been reached as result of the intuition that an element realised in a shape memory material, in the passage from the transient shape towards the working shape, can create the fixing of the intramedullary nail in the bone.

Expansion means 20 of the proximal end 16 comprise a cylindrical sleeve 22 which is worn around the stem 14. The cylindrical sleeve 22, undergone an axial compression, buckles taking a cask configuration, outside the stem 14, even taking an almost spherical configuration.

Expansion means 21 of the distal end 18 comprise a cylindrical sleeve 23 which is worn around the stem 14. Also the cylindrical sleeve 23, undergone an axial compression, buckles taking a cask configuration, outside the stem 14, even taking an almost spherical configuration.

Said at least one driving element 30 is interposed between the cylindrical sleeve 22 and the cylindrical sleeve 23. The driving element 30 comprises an intermediate cylindrical sleeve 31, worn around the stem 14, with a radial dimension being substantially equal to the radial dimension of the two sleeves 22 and 23 and realised with a shape-memory material: in the final shape, causing the fixation of the nail 10 to the bone 12, said intermediate cylindrical sleeve 31 undergoes an extension.

The stem 14 has at the distal end 18 or at the proximal end 16 an thickening 28, a front end of the cylindrical sleeve 23 or a front end of the cylindrical sleeve 22 respectively abutting on a surface thereof. The intermediate cylindrical sleeve 31 and the cylindrical sleeve 22 (or the cylindrical sleeve 23 respectively) are then mounted on the stem 14, said two cylindrical sleeves 22 and 23 abutting on the two front ends of the intermediate cylindrical sleeve 31.

The stem 14 has, in correspondence with the proximal end 16 or the distal end 18 respectively, a thread wherein a retaining ring 29 of the series of the first 22, second 23 and intermediate cylindrical sleeve 31 is screwed. Preferably, the ring 29, which is realised for example with a titanium alloy, is screwed on the stem 14 up to exert a slight pressure on the series of the sleeves 22, 23 and 31, i.e. said sleeves 22, 23 and 31 are positioned with an axial preload.

In order to screw the ring 29, two or more side flats (preferably parallel at a calibrated distance) are provided thereon for using a suitable control spanner.

At the top of the stem 14, on the side wherein the ring 29 is mounted, for example an hexagon-shaped profile is advantageously obtained, to fasten the stem 14 (with respect to the ring 29) by means of a spanner for socket hexagonal head.

The intermediate cylindrical sleeve 31 undergoes an instruction step causing it, in the final shape, to axially extend: this deformation causes on the first sleeve 21 and on the second sleeve 23 said axial compression buckling the first 22 and second 23 sleeve as above described.

In an alternative embodiment, the intermediate cylindrical sleeve 31 is realised with a titanium alloy, while the driving element 30 is composed of the stem 14, which is realised with a shape-memory material: in the final shape, causing the fixation of the nail 10 to the bone 12, said stem 14 undergoes a shortening. In other words, the stem 14 undergoes an instruction step causing it, in the final shape, to axially shorten: this deformation causes on the first sleeve 22 and on the second sleeve 23 said axial compression buckling the first 22 and second 23 sleeve as above described.

According to a further alternative embodiment of the invention, the two cylindrical sleeves 22 and 23 are realised with a shape-memory material: said cylindrical sleeves 22 and 23 undergo an instruction step causing them, in the final shape, to take a cask configuration, outside the stem 14, even taking an almost spherical configuration.

Therefore, in this alternative embodiment, both expansion means 20 and 21, and the intermediate cylindrical sleeve 31 are realised with shape-memory materials. The intermediate cylindrical sleeve 31 undergoes an instruction step causing it, in the final shape, to axially extend: this deformation causes on the first sleeve 22 and on the second sleeve 23 a compression load tending to further increase the deformation of the first 22 and second sleeve 23.

Referring now to figures 2 and 3, a second embodiment of intramedullary nail is shown, according to the present invention, and globally indicated with 110, suitable for insertion in a fractured elongate bone 112. In this embodiment, the elements being structurally or functionally similar to the elements of the intramedullary nail 10 are indicated with the same reference number increased by 100 and the detailed description thereof is not repeated hereafter.

The nail 110 comprises a substantially straight stem 114 extending between a proximal end 116 and a distal end 118.

The stem 114 is preferably composed of a cylindrical tube, realised for example with a titanium alloy. The stem 114, according to an aspect of the present invention, comprises, near each of said proximal 116 and distal 118 ends, expansion means 120 and 121 respectively for the fixation of the nail 110 to the bone 112, said expansion means 120 and 121 being operated by at least one driving element 130, interposed between said expansion means 120 of said proximal end 116 and said expansion means 121 of said distal end 118 and realised with a shape-memory material.

Expansion means 120 and 121 of the proximal 116 and distal ends are of a same type; they comprise a plurality of inserts 122 and 123, sliding in corresponding openings 124 and 125 provided around the cylindrical sleeve of the stem 114 between an initial position, wherein an upper end 122a and 123a of the inserts 122 and 123 is substantially positioned below or on the outer side surface of the stem 114, and a final position, wherein the upper end 122a and 123a of the inserts 122 and 123 is positioned outside the outer side surface of the stem 114. The final position of said inserts 122 and 123 is defined by striker elements 122b and 123b abutting on a surface of the stem 114 positioned around the openings 124 and 125, inside the stem 114.

The upper end 122a and 123a of the inserts 122 and 123 is, for example, flat or slightly convex.

A flare 122c and 123c respectively is also preferably realised above the inserts 122 and 123, for example countersunk at 45°, towards the central part of the stem 114.

A housing groove 122d and 123d respectively of an elastic clamping ring (not shown in the figures) is also provided above the inserts 122 and 123, joining together the plurality of inserts 122 and 123 respectively.

The inserts 122 and 123 have a lower sloping surface 126 and 127, preferably flat, sloping from the side positioned towards the two ends of the stem 114 respectively.

Each of the expansion means 120 and 121 also comprises a driving element 132 and 133 to bring said inserts 124 and 125 in said final position: each of said driving elements 132 and 133 slides in the cylindrical sleeve of the stem 114 and it is equipped with a plurality of sloping surfaces, sloping from the opposite side with respect to the two respective ends of the stem 114, each of said sloping surfaces being conjugated to a corresponding lower sloping surface 126 and 127 of the inserts 122 and 123.

More precisely, the plurality of sloping surfaces of the driving elements 132 and 133 is realised, in the example of the figures, with a single frustum conical surface 134 and 135 respectively.

Said at least one driving element 130 is interposed between the two driving elements 132 and 133, in the cylindrical sleeve of the stem 114. The driving element 130 comprises a spring 131 realised with a shape-memory material: in the final shape, causing the fixation of the nail 110 to the bone 112, said spring 131 undergoes an extension along the direction of the axis of the stem 114.

Preferably, the inserts 122 and 123 and the driving elements 132 and 133 are realised with a titanium alloy.

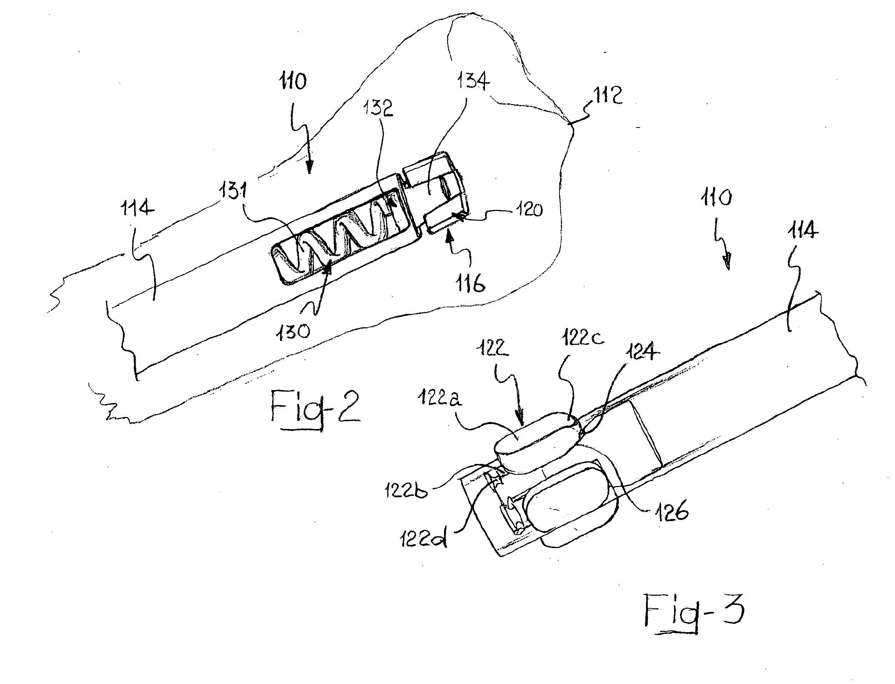

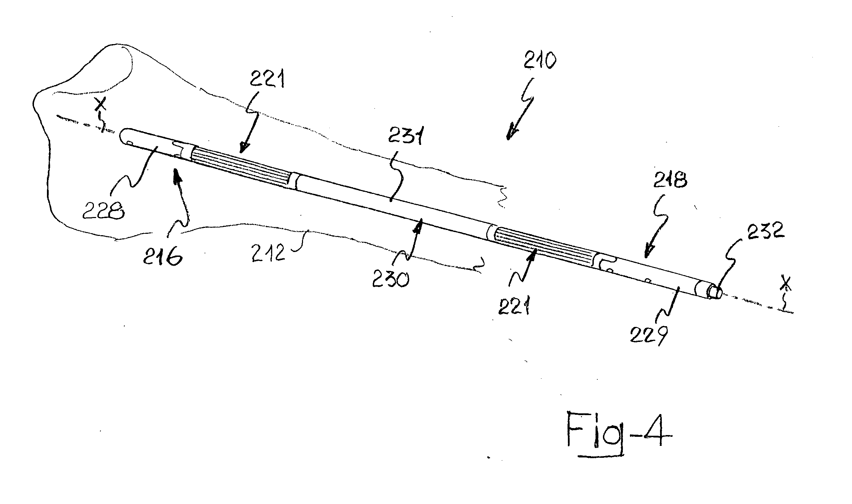

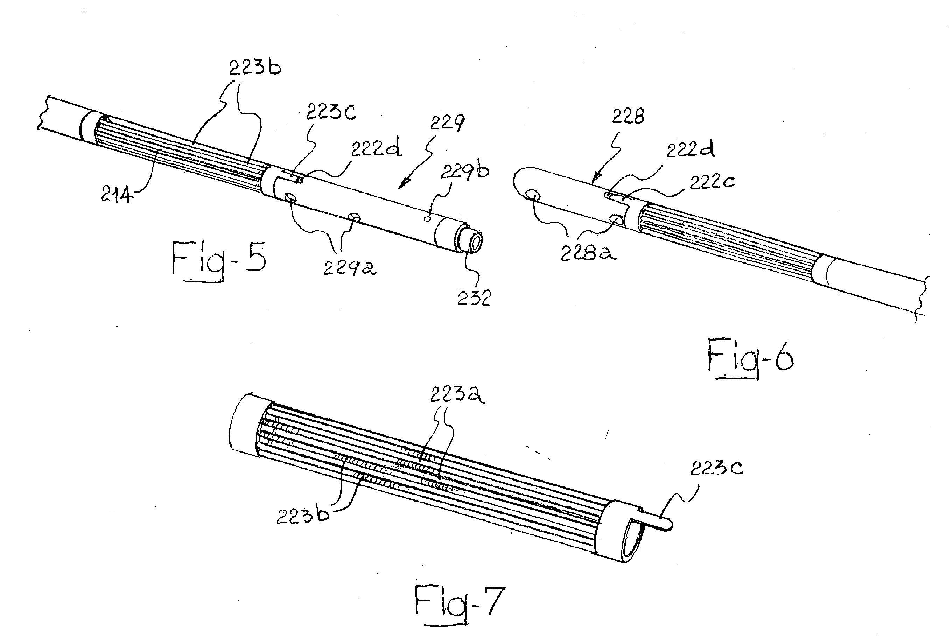

Referring now to figures 4, 5, 6 and 7, a third embodiment of intramedullary nail is shown, according to the present invention, and globally indicated with 210, suitable for insertion in a fractured elongate bone 212. In this embodiment, the elements being structurally or functionally similar to the elements of the intramedullary nail 10 are indicated with the same reference number increased by 200 and the detailed description thereof is not repeated hereafter.

The nail 210 comprises a substantially straight stem 214 extending between a proximal end 216 and a distal end 218.

The stem 214 is preferably composed of a cylindrical tube, realised for example with a titanium alloy. The stem 214, according to an aspect of the present invention, comprises, near each of said proximal 216 and distal 218 ends, expansion means 220 and 221 respectively for the fixation of the nail 210 to the bone 212, said expansion means 220 and 221 being operated by at least one driving element 230, interposed between said expansion means 220 of said proximal end 216 and said expansion means 221 of said distal end 218 and realised with a shape-memory material.

The expansion means 220 of the proximal end 216 comprise a cylindrical sleeve 222 which is worn around the stem 214. The cylindrical sleeve 222 preferably comprises a plurality of longitudinal slots 222a, for example equally spaced around the cylinder of the first sleeve 222, the slots 222a defining a plurality of staves 222b.

Moreover, the first sleeve 222 comprises at least one first tongue-shaped appendix 222c extending, in a longitudinal direction, outside the stem 214: the appendix 222c is housed in a corresponding first tongue housing 222d provided on an thickening 228 of the stem 214, realised at the proximal end 216 of the stem 214, beyond the cylindrical sleeve 222.

The thickening 228, having a cylindrical shape and a radial dimension being substantially equal to the dimension of the cylindrical sleeve 222, preferably comprises holes 228a for bone screws, for example two.

In the final shape, causing the fixation of the nail 210 to the fractured bone 212, said cylindrical sleeve 222, undergoing an axial compression, takes a cask configuration outside the stem 214, with a subsequent shortening of the longitudinal dimension of the sleeve 222; in the preferred embodiment with the staves 222b, in the final shape, the staves 222b take the cask stave configuration, with a subsequent shortening of the longitudinal dimension of the sleeve 222.

The first tongue-shaped appendix 222c, when passing from the initial shape to the final shape, slides in the first tongue housing 222d and also in said final shape the first tongue-shaped appendix 222c is inserted in the corresponding tongue housing 222d.

The expansion means 221 of the proximal end 218 comprise a cylindrical sleeve 223 which is worn around the stem 214. The cylindrical sleeve 223 preferably comprises a plurality of longitudinal slots 223a, for example equally spaced around the cylinder of the first sleeve 223, the slots 223a defining a plurality of staves 223b.

Moreover, the first sleeve 223 comprises at least one second tongue-shaped appendix 223c extending, in a longitudinal direction, outside the stem 214: the appendix 223c is housed in a corresponding second tongue housing 223d provided on an thickening 229 of the stem 214, realised at the proximal end 218 of the stem 214, beyond the cylindrical sleeve 223.

The thickening 229, having a cylindrical shape and a radial dimension being substantially equal to the dimension of the cylindrical sleeve 223, preferably comprises holes 229a for bone screws, for example two.

In the final shape, causing the fixation of the nail 210 to the fractured bone 212, said cylindrical sleeve 223, undergoing an axial compression, takes a cask configuration outside the stem 214, with a subsequent shortening of the longitudinal dimension of the sleeve 223; in the preferred embodiment with the staves 223b, in the final shape, the staves 223b take the cask stave configuration, with a subsequent shortening of the longitudinal dimension of the sleeve 223.

The second tongue-shaped appendix 223c, when passing from the initial shape to the final shape, slides in the second tongue housing 223d and also in said final shape the second tongue-shaped appendix 223c is inserted in the corresponding tongue housing 223d.

Said at least one driving element 230 is interposed between the cylindrical sleeve 222 and the cylindrical sleeve 223. The driving element 230 comprises an intermediate cylindrical sleeve 231, worn around the stem 214, with a radial dimension being substantially equal to the radial dimension of the two sleeves 222 and 223 and realised with a shape-memory material: in the final shape, causing the fixation of the nail 210 to the bone 212, said intermediate cylindrical sleeve 231 undergoes an extension.

Preferably, as shown in the example of the figures, the thickening 229 comprises a cylindrical sleeve and the distal end 218 of the stem 214 has a thread. A retaining ring 232 of the series of the first 222, second 231 and cylindrical sleeve 223 is screwed on said thread. Preferably, the ring 232 is screwed on the stem 214 up to exert a slight pressure on the series of the sleeves 222, 223 and 231, i.e. said sleeves 222, 223 and 231 are positioned with an axial preload. In this shape, the cylindrical sleeve of the thickening 229 is fixed to the stem 214, for example by positioning, in a radial direction, a pin 229b between the sleeve and the stem 214.

The intermediate cylindrical sleeve 231 undergoes an instruction step causing it, in the final shape, to axially extend: this deformation causes on the first sleeve 222 and on the second sleeve 223 said axial compression buckling the first 222 and second sleeve 223 as above described.

According to an alternative embodiment of the invention, the two cylindrical sleeves 222 and 223 are realised, at least partially, with a shape-memory material: said cylindrical sleeves 222 and 223 undergo an instruction step causing them, in the final shape, to take a cask configuration, outside the stem 214.

More precisely, the staves 222b and 223b are realised with a shape-memory material. By way of example, nitinol can be used, which, as it is well known, is a nickel-titanium alloy created in 1962 by Buehler, a metallurgist at the U.S. Naval Ordinance Laboratory. The staves 222b and 223b can thus comprise nitinol fillets.

Instead of nitinol, a superelastic material can be used, i.e. allowing considerable constant-load deformations: such a material is a nickel-titanium alloy and it differs from nitinol only for the percentages of these components.

It must be underlined that the staves 222b and 223b can also comprise wires made of nitinol or other shape-memory material, instead of said nitinol fillets.

Therefore, in this alternative embodiment, both the expansion means 220 and 221 and the intermediate cylindrical sleeve 231 are, at least partially, realised with shape-memory materials. The intermediate cylindrical sleeve 231 undergoes an instruction step causing it, in the final shape, to axially extend: this deformation causes on the first sleeve 222 and on the second sleeve 223 a compression load tending to further increase the deformation of the first 222 and second sleeve 223.

It is specified that, in the third embodiment of the nail 210 of the invention, the positions of the components of the nail 210 can be obviously inverted with respect to the proximal 216 and distal 218 ends of the stem 214, in the sense that the ring 232 can be positioned at the proximal end 216 rather than at the distal end 218.

An application method, according to the present invention, of said intramedullary nail in said elongate bone, comprises:
a location step of said nail in said elongate bone, to mend the fracture;
an operation step of said expansion means, provided at each of the proximal and distal ends of the stem, for the nail fixation to the bone, said expansion means being operated by at least a driving element realised with a shape-memory material.

The operation of the intramedullary nail, suitable for insertion in a fractured elongate bone, according to the present invention, is already partially evident from the description of the above application method and it is specified hereafter.

In the case of the first embodiment of the intramedullary nail 10, the nail 10 is positioned in said elongate bone 12, in the initial shape thereof, with the two cylindrical sleeves 22 and 23 having a cylindrical shape.

Afterwards, in order to fix the nail 10 to the bone 12,' said expansion means 20 and 21 are operated through the extension of the intermediate cylindrical sleeve 31, so that the two cylindrical sleeves 22 and 23 tend to bend outside the stem 14, with a subsequent interference of the sleeves 22 and 23: this interference practically causes a grip, solidly fixing said nail 10 in the bone 12.

It is worth noting, once and for all, that the cylindrical tube shape of the stem has a double function: in fact it allows the stem to be inserted on a guide wire and it allow the elements realised with a shape-memory material to be heated or cooled by means of a probe being inserted in the cylindrical tube.

In the alternative embodiment wherein the two cylindrical sleeves 22 and 23 are realised with a shape-memory material, the extension of the intermediate cylindrical sleeve 31 increases the deformation of the two cylindrical sleeves 22 and 23, which per se already tend to bend outside the stem 14, with a subsequent increased interference of the sleeves 22 and 23 with respect to the bone portion surrounding said sleeves 22 and 23.

The structural function of the nail 10 is provided by the titanium stem 14.

It must be observed that the compression stress generated by the extension of the driving element 30 creates, by friction, a considerable torsional resistance between the different parts of the nail 10 (particularly, between the stem 14 and the two cylindrical sleeves 22 and 23), so that the nail 10 does not require particular torsional control means.

By using a cylindrical sleeve 31 having a considerable length, it must be noticed that it is possible to realise large deformations of the expansion means 20 and 21. Advantageously, standardised cylindrical sleeves 31 with different lengths can be realised: they are modular to each other, up to obtain the desired length for the set of the driving elements 30 used in the nail 10.

In the case of the alternative embodiment wherein the intermediate cylindrical sleeve 31 is realised with a titanium alloy and the driving element 30 is composed of the stem 14, for the fixation of the nail to the bone 12 said expansion means 20 and 21 are operated by shortening the stem 14, so that the two cylindrical sleeves 22 and 23 tend to bend outside the stem 14. In this case the intermediate cylindrical sleeve 31 serves as a spacer.

In the case of the second embodiment of the intramedullary nail 110, the nail 110 is positioned in said elongate bone 112, in the initial shape thereof, with the upper ends 122a and 123a of the inserts 122 and 123 being positioned substantially below or on the outer side surface of the stem 114.

Afterwards, in order to fix the nail 110 to the bone 112, the spring 131 undergoes and extension along the axis direction of the stem 114, so that the driving elements 132 and 133 are axially moved towards the respective ends of the stem 114: the upper ends 122a and 123a of the inserts 122 and 123 thus tend to project from the outer side surface of the stem 114, with a subsequent compression of the inserts 122 and 123 with respect to the bone portion surrounding said inserts 122 and 123: this compression practically causes a grip, solidly fixing said nail 110 in the bone 112.

It must be underlined that, in the preferred alternative embodiment, the flares 122c and 123c of the inserts 122 and 123 help the inserts 122 and 123 in returning in respective openings 124 and 125 in a possible extraction of the nail 110. To this purpose, during this extraction the reversibility of the motion of the driving elements 132 and 133 and inserts 122 and 123 must be allowed: the frustum conical surface 134 and 135 of the driving elements 132 and 133 has thus for example a slope of about 45° with respect to the axis of the stem 114.

It is also specified that the grooves 122d and 123d serve to position a respective elastic ring holding the plurality of inserts 122 and 123 respectively in a closed position, before the intervention of the respective driving element 132 and 133 to bring said inserts 124 and 125 in the final position.

Finally, it must be observed that the structural function of the nail 110 is provided by the stem 114 and that the spring 131 ensures a considerable deformation and an excellent compression load on the driving elements 132 and 133.

In the case of the third embodiment of the intramedullary nail 210, the nail 210 is positioned in said elongate bone 212, in the initial shape thereof, with the two cylindrical sleeves 222 and 223 being cylinder-shaped.

Afterwards, in order to fix the nail 210 to the bone 212, said expansion means 220 and 221 are operated through the extension of the intermediate cylindrical sleeve 231, so that the two cylindrical sleeves 222 and 223 tend to bend outside the stem 214, with a subsequent interference of the sleeves 222 and 223 with respect to the bone portion surrounding said sleeves 222 and 223: this interference practically causes a grip, solidly fixing said nail 210 in the bone 212.

In the alternative embodiment wherein the two cylindrical sleeves 222 and 223 are at least partially realised with a shape-memory material, the extension of the intermediate cylindrical sleeve 231 increases the deformation of the two cylindrical sleeves 222 and 223, which per se already tend to bend outside the stem 214, with a subsequent increased interference of the sleeves 222 and 223 with respect to the bone portion surrounding said sleeves 222 and 223.

The shape-memory materials being used can be for example operated by the human body inner temperature, so that, once the nail 210 is positioned in the bone 212, there is nothing but waiting that the shape-memory components reach such a temperature.

An alternative method for operating the shape-memory materials being used is to insert, in the cylindrical tube of the stem 214, a probe whose temperature is set during the surgical operation.

The structural function of the nail 210 is provided by the stem 214 and by the thickenings 228 and 229.

The pin 229b serves to prevent undesired torsions or rotations between the stem 214 and the cylindrical sleeve of the thickening 229.

It must be underlined that the use of nitinol as shape-memory material ensure a constant load with respect to the bone.

Moreover, the particular kind of clamping by means of the retaining ring 232 allows a single-way shape-memory material to be used, i.e. a material in which the operation step occurs only once and it is impossible, once the operation conditions have been set, to return to the initial shape. In fact, by means of the ring 232, the extraction of the nail 210 occurs by unscrewing the ring 232 and discharging thus the axial load from the two cylindrical sleeves 222 and 223.

In the suggested solution two holes 228a and two holes 229a for bone screws are provided. The surgeon can thus decide to put one or two bone screws, or he can decide to put no screws, according to the clinical cases occurring to him.

It must be finally noticed that the tongue-shaped appendixes 222c and 223c ensure the possibility to locate the holes 228a and 229a, knowing the mutual positions of the tongue housings 222d and 223d: this is obtained by using a suitable template.

The main advantage achieved by the intramedullary nail suitable for insertion in a fractured elongate bone, as well as by the application method of said nail in said bone, according to the present invention, is the fact of unusually simplifying the fixation step of the intramedullary nail inserted in the bone. In practise, the nail fixation is obtained simultaneously with the insertion thereof in the bone, since the shape-memory elements, cooled at first to take the initial shape, take in the bone their final shape, due to the heat released by the patient's body.

Another advantage of the intramedullary nail suitable for insertion in a fractured elongate bone, according to the present invention, is to prevent undesired nail torsions or rotations.

Obviously, in order to meet specific and contingent requirements, a skilled in the art could bring several changes to the above-described intramedullary nail suitable for insertion in a fractured elongate bone and application method of said nail in said bone, all however comprised in the scope of protection of the present invention, as defined in the following claims.

## Claims

1. Intramedullary nail (10, 110, 210) suitable for insertion in a fractured elongate bone (12, 112, 212), comprising a substantially straight stem (14, 114, 214) extending between a proximal end (16, 116, 216) and a distal end (18, 118, 218), **characterised in that** said stem (14, 114, 214) comprises, near each of said proximal (16, 116, 216) and distal (18, 118, 218) ends, expansion means (20, 120, 220; 21, 121, 221) for the nail fixation to the bone, said expansion means (20, 120, 220; 21, 121, 221) being operated by at least one driving element (30, 130, 230), interposed along the axis of the stem (14, 114, 214) between said expansion means (20, 120, 220) of said proximal end (16, 116, 216) and said expansion means (21, 121, 221) of said distal end (18, 118, 218) and realised with a shape-memory material.

2. Intramedullary nail (10, 210) according to claim 1, **characterised in that** said expansion means (20, 220) of the proximal end (16, 216) comprise a cylindrical sleeve (22, 222) which is worn around the stem (14, 214), the cylindrical sleeve (22, 222), undergoing an axial compression, buckling and taking a cask configuration, outside the stem (14, 214).

3. Intramedullary nail (10, 210) according to claim 2, **characterised in that** said expansion means (21, 221) of the distal end (18, 218) comprise a cylindrical sleeve (23, 223) which is worn around the stem (14, 214), the cylindrical sleeve (23, 223), undergoing an axial compression, buckling and taking a cask configuration, outside the stem (14, 214).

4. Intramedullary nail (10) according to claim 3, **characterised in that**, said at least one driving element (30) is interposed between the two cylindrical sleeve (22, 23), the driving element (30) comprising an intermediate cylindrical sleeve (31), worn around the stem (14), with a radial dimension being substantially equal to the radial dimension of the first (22) and second sleeve (23), in the final shape, causing the fixation of the nail (10) to the bone (12), said intermediate cylindrical sleeve (31) undergoing an extension.

5. Intramedullary nail (10) according to claim 4, **characterised in that** the stem (14) has at the distal end (18) a thickening (28), a front end of the cylindrical sleeve (23) of the distal end (18) abutting on a surface thereof, the intermediate cylindrical sleeve (31) and the cylindrical sleeve (22) of the proximal end (16) being then mounted on the stem (14), said two cylindrical sleeves (22, 23) thus abutting on the two front ends of the intermediate cylindrical sleeve (31).

6. Intramedullary nail (10) according to claim 4, **characterised in that** the stem (14) has at the proximal end (16) a thickening (28), a front end of the cylindrical sleeve (22) of the proximal end (16) abutting on a surface thereof, the intermediate cylindrical sleeve (31) and the cylindrical sleeve (23) of the distal end (18) being then mounted on the stem (14), said two cylindrical sleeves (22, 23) thus abutting on the two front ends of the intermediate cylindrical sleeve (31).

7. Intramedullary nail (10) according to claims 5 or 6, **characterised in that** the stem (14) has, in correspondence with a proximal (16) or distal (18) end respectively, a thread wherein a retaining ring (29) of the series of the first (22), second (23) and intermediate cylindrical sleeve (31) is screwed.

8. Intramedullary nail (10) according to claim 7, **characterised in that** said ring (29) is screwed on the stem (14) up to exert a slight pressure on the series of the sleeves (22, 23, 31).

9. Intramedullary nail (10) according to claim 7, **characterised in that** said ring (29) is realised with a titanium alloy.

10. Intramedullary nail (10) according to claim 7, **characterised in that** two or more side flats are provide on said ring (29) for using a suitable control spanner.

11. Intramedullary nail (10) according to claim 7, **characterised in that** said two side flats are parallel and at a calibrated distance.

12. Intramedullary nail (10) according to claim 7, **characterised in that**, at the top of the stem (14), on the side wherein the ring (29) is mounted, an hexagon-shaped profile is obtained.

13. Intramedullary nail (10) according to claim 5 or 6, **characterised in that** the two cylindrical sleeves (22, 23) are realised with a shape-memory material, said two cylindrical sleeves (22, 23), in said final shape, taking a cask configuration, outside the stem (14).

14. Intramedullary nail (10) according to claim 3, **characterised in that**, an intermediate cylindrical sleeve (31) is interposed between the two cylindrical sleeves (22, 23), worn around the stem (14), with a radial dimension being substantially equal to the radial dimension of the first (22) and second sleeve (23) and realised with a titanium alloy, said at lest one driving element (30) being composed of said stem (14), realised with a shape-memory material, in the final shape, causing the fixation of the nail (10) to the bone (12), said stem (14) undergoing a shortening.

15. Intramedullary nail (110) according to claim 1, **characterised in that** the stem (114) is composed of a cylindrical tube and **in that** said expansion means (120, 121) of the proximal (116) and distal end respectively comprise a plurality of inserts (122, 123), sliding in corresponding openings (124, 125) provided around the cylindrical sleeve of the stem (114) between an initial position, wherein an upper end (122a, 123a) of the inserts (122, 123) is positioned substantially below or on the outer side surface of the stem (114), and a final position, wherein the upper end (122a, 123a) of the inserts (122, 123) is positioned outside the outer side surface of the stem (114).

16. Intramedullary nail (110) according to claim 15, **characterised in that** the final position of said inserts (122, 123) is defined by striker elements (122b, 123b) abutting on a surface of the stem (114) positioned around the openings (124, 125), in the stem (114).

17. Intramedullary nail (110) according to claim 15, **characterised in that** the upper end (122a, 123a) of the inserts (122, 123) is flat.

18. Intramedullary nail (110) according to claim 15, **characterised in that** the upper end (122a, 123a) of the inserts (122, 123) is slightly convex.

19. Intramedullary nail (110) according to claim 15, **characterised in that** a flare (122c, 123c) is realised above the inserts (122, 123) towards the central part of the stem (114).

20. Intramedullary nail (110) according to claim 19, **characterised in that** the flare (122c, 123c) is countersunk 45°.

21. Intramedullary nail (110) according to claim 15, **characterised in that** a housing groove (122d, 123d) of an elastic clamping ring is realised above the inserts (122, 123), joining together each plurality of inserts (122, 123).

22. Intramedullary nail (110) according to claim 15, **characterised in that** the inserts (122, 123) have a lower sloping surface (126, 127), sloping from the side positioned towards the two ends (116, 118) of the stem (114).

23. Intramedullary nail (110) according to claim 22, **characterised in that** said lower sloping surface (126, 127) is flat.

24. Intramedullary nail (110) according to claim 15, **characterised in that** each of the expansion means (120, 121) comprises a driving element (132, 133) to bring said inserts (124, 125) in said final position.

25. Intramedullary nail (110) according to claims 22 and 24, **characterised in that** each of said driving elements (132, 133) slides in a cylindrical sleeve of the stem (114) and it is equipped with a plurality of sloping surfaces, sloping from the opposite side with respect to the two ends (116, 118) of the stem (114), each of said sloping surfaces being conjugated to a corresponding lower sloping surface (126, 127) of the inserts (122, 123).

26. Intramedullary nail (110) according to claim 25, **characterised in that** said plurality of sloping surfaces of the driving elements (132, 133) is realised with a single frustum conical surface (134, 135).

27. Intramedullary nail (110) according to claim 25, **characterised in that** said at least one driving element (130) is interposed between the two driving elements (132, 133), in the cylindrical sleeve of the stem (114), the driving element (130) comprising a spring (131) realised with a shape-memory material: in the final shape, causing the fixation of the nail (110) to the bone (112), said spring (131) undergoing an extension along the direction of the axis of the stem (114).

28. Intramedullary nail (110) according to claim 24, **characterised in that** the inserts (122, 123) and the driving elements (132, 133) are realised with a titanium alloy.

29. Intramedullary nail (210) according to claim 1, **characterised in that** said expansion means (220) of the proximal end (216) comprise a cylindrical sleeve (222) which is worn around the stem (214), the cylindrical sleeve (222) comprising a plurality of longitudinal slots (222a).

30. Intramedullary nail (210) according to claim 29, **characterised in that** said slots (222a) are equally spaced around the cylinder of the first sleeve (222), the slots (222a) defining a plurality of staves (222b).

31. Intramedullary nail (210) according to claim 29, **characterised in that** the first sleeve (222) comprises at least a first tongue-shaped appendix (222c) extending, in longitudinal direction, outside the stem (214), the appendix (222c) being housed in a corresponding first tongue housing (222d) provided on a thickening (228) of the stem (214), realised at the proximal end (216) of the stem (214), beyond the cylindrical sleeve (222).

32. Intramedullary nail (210) according to claim 31, **characterised in that** said thickening (228), having a cylindrical shape and a radial dimension being substantially equal to the dimension of the cylindrical sleeve (222), preferably comprises holes (228a) for bone screws.

33. Intramedullary nail (210) according to claim 30, **characterised in that** the staves (222b), in the final shape, causing the fixation of the nail (210) to the bone (212), take cask stave configuration.

34. Intramedullary nail (210) according to claim 31, **characterised in that** the first tongue-shaped appendix (222c) slides in the first tongue housing (222d) and **in that** in said final shape the first tongue-shaped appendix (222c) is inserted in the first tongue housing (222d).

35. Intramedullary nail (210) according to claim 29, **characterised in that** said expansion means (221) of the distal end (218) comprise a cylindrical sleeve (223) which is worn around the stem (214), the cylindrical sleeve (223) comprising a plurality of longitudinal slots (223a).

36. Intramedullary nail (210) according to claim 35, **characterised in that** said slots (223a) are equally spaced around the cylinder of the first sleeve (223), the slots (223a) defining a plurality of staves (223b).

37. Intramedullary nail (210) according to claim 35, **characterised in that** the second sleeve (223) comprises at least a second tongue-shaped appendix (223c) extending, in longitudinal direction, outside the stem (214), the appendix (223c) being housed in a corresponding second tongue housing (223d) provided on a thickening (229) of the stem (214), realised at the distal end (218) of the stem (214), beyond the cylindrical sleeve (223).

38. Intramedullary nail (210) according to claim 37, **characterised in that** said thickening (229), having a cylindrical shape and a radial dimension being substantially equal to the dimension of the cylindrical sleeve (223), preferably comprises holes (229a) for bone screws.

39. Intramedullary nail (210) according to claim 36, **characterised in that** the staves (223b), in the final shape, causing the fixation of the nail (210) to the bone (212), take cask stave configuration.

40. Intramedullary nail (210) according to claim 37, **characterised in that** the second tongue-shaped appendix (223c) slides in the second tongue housing (223d) and **in that** in said final shape the second tongue-shaped appendix (223c) is inserted in the second tongue housing (223d).

41. Intramedullary nail (210) according to claim 35, **characterised in that** said at least one driving element (230) is interposed between the two cylindrical sleeves (222,223), the driving element (230) comprising an intermediate cylindrical sleeve (231), worn around the stem (214), with a radial dimension being substantially equal to the radial dimension of the first (222) and second sleeve (223), in the final shape, causing the fixation of the nail (210) to the bone (212), said intermediate cylindrical sleeve (231) undergoing an extension.

42. Intramedullary nail (210) according to claim 37, **characterised in that** the thickening (229) comprises a cylindrical sleeve and the distal end (218) of the stem (214) has a thread, a retaining ring (232) of the series of the cylindrical sleeve (222) of the proximal end (216), of the further cylindrical sleeve (231) and of the cylindrical sleeve (223) of the distal end (218) being screwed on said thread.

43. Intramedullary nail (210) according to claim 42, **characterised in that** the ring (232) is screwed on the stem (214) up to exert a slight pressure on the series of the sleeves (222, 223, 231).

44. Intramedullary nail (210) according to claim 43, **characterised in that** the cylindrical sleeve of the thickening (229) is fixed to the stem (214) positioning in a radial direction a pin (229b) between the sleeve and the stem (214).

45. Intramedullary nail (210) according to claims 41 or 43, **characterised in that** the two cylindrical sleeves (222, 223) are at least partially realised with a shape-memory material, said two cylindrical sleeves (222, 223), in said final shape, taking a cask configuration, outside the stem (214).

46. Intramedullary nail (210) according to claims 30, 36 and 45, **characterised in that** the staves (222b, 223b) are realised with a shape-memory material.

47. Intramedullary nail (210) according to claims 30, 36 and 45, **characterised in that** the staves (222b, 223b) are realised with a superelastic material, i.e. allowing considerable constant load deformations.

48. Intramedullary nail (210) according to claim 46, **characterised in that** the staves (222b, 223b) comprise nitinol fillets.

49. Intramedullary nail (210) according to claim 45, **characterised in that** the staves (222b, 223b) comprise nitinol wires.

50. Intramedullary nail (10, 210) according to claim 1, in **characterised in that** the stem (14, 214) is composed of a cylindrical tube.

51. Intramedullary nail (10, 110, 210) according to claim 1, **characterised in that** the stem (14, 114, 214) is realised with a titanium alloy.

52. Intramedullary nail (10, 110, 210) according to claim 1, **characterised in that** said fractured elongate bone (12, 112, 212) is a femur or a tibia.

53. Application method, in a fractured elongate bone (12, 112, 212), of an intramedullary nail (10, 110, 210) according to claim 1, **characterised in that** it comprises:
a positioning step of said nail (10, 110, 210) in said elongate bone (12, 112, 212), to mend the fracture;
an operation step of said expansion means (20, 120, 220; 21, 121, 221) provided at each of the proximal (16, 116, 216) and distal (18, 118, 218) ends of the stem (14, 114, 214), for the nail fixation to the bone, said expansion means (20, 120, 220; 21, 121, 221) being operated by at least one driving element (30, 130, 230) realised with a shape-memory material.
